# EUROPEAN PATENT SPECIFICATION

(11) **EP 0 308 675 B1**
(45) Date of publication and mention of the grant of the patent: **03.02.1993**
(21) Application number: 88113867.1
(22) Date of filing: 25.08.1988
(51) Int. Cl.: B01D 11/02

(54) **Process for the supercritical extraction and separation of solid samples**
Verfahren zur Extraktion und Trennung von festen Mustern unter überkritischen Bedingungen
Procédé d'extraction et de séparation des échantillons solides sous des conditions supercritiques

(30) Priority: 07.09.1987 JP 222022/87
(43) Date of publication of application: 29.03.1989
(73) Proprietor: CHLORINE ENGINEERS CORP., Ltd., Tokyo 105 (JP); KABUSHIKI KAISHA YAKULT HONSHA, Minato-ku Tokyo 105 (JP)
(72) Inventor: Manabe, Akiyoshi, Fujisawa-shi Kanagawa 251 (JP); Yamashita, Tetsuro, Minato-ku Tokyo 105 (JP); Harada, Katsuhisa, Minato-ku Tokyo 105 (JP); Tokumori, Tsuneo, Kurashiki-shi Okayama 704-11 (JP); Sumida, Yoko, Okayama-shi Okayama 700 (JP)
(74) Representative: Klingseisen, Franz, Dipl.-Ing.

(56) References cited:
- EP-A- 0 086 315
- EP-A- 0 154 258
- EP-A- 0 252 394
- DE-A- 2 639 066

## Description

The present invention relates to a process according to the preamble of claim 1.

DE-OS 26 39 066 discloses a process for extracting and separating substances in coffee beans by using a fluid in supercritical state as a solvent. The fluid in supercritical state used is carbon dioxide, which is contacted with the coffee beans. The coffee beans contain a certain content of water and are piled up in an extractor. Said dissolving process can be improved by entrainers, e.g. methanol, ethanol, which are added to the supercritical fluid. A certain content of water is controlled preferably to 2 to 60 % and more desirably to 20 to 35 %. After the extraction process the beans have to undergo a well-known drying process.

Further, the EP-OS 0 154 258 describes a process for extracting different spices from different parts of plants with, for instance, supercritical CO₂ with ethanol as entrainer. This publication shows how an entrainer is used for selectively extracting predetermined spices.

For the extraction of effective components or removal of harmful components from, e.g., naturally occurring materials, there has been employed steam distillation or solvent extraction utilizing such solvents as esters, hydrocarbons, halogenated hydrocarbons, or the like.

There are, however, cases where the use of organic solvents is restricted. For example, in cases where extracts from naturally occurring materials are to be used for foods or medical purposes, organic solvents may not be employed for their extraction, or extracts obtained must be subjected to aftertreatment in order to reduce the quantity of organic solvents remaining therein.

In view of the above, extraction processes utilizing supercritical carbon dioxide have been employed in German Offenlegungsschrift No. 2212281 ("Extraction of Caffeine from Coffee") and in German Offenlegungsschrift No. 2127642 ("Production of Caffein-free Tea Extracts").

The present inventors have conducted intensive investigations with the purpose of applying extraction with a supercritical fluid, in particular, carbon dioxide (which is safe enough to be permitted as a food additive) to the extraction and purification of effective components contained in ginkgo leaves, and have accomplised the present invention.

Ginkgo is a deciduous tree of which original habitat is China. Giant ginkgo trees are seen in many places. Ginkgo is known as one of the oldest plants which are found in fossils and still exist. Ginkgo trees are strongly resistant to blight and noxious insects. The trees contain various useful substances, and the kernel (albumen and embryo) of ginkgo seeds gas been utilized as food from the ancient time.

In particular, leaves of ginkgo contain vaso-active substances capable of expanding capillary and such substances have hitherto been extracted and purified with organic solvents.

In addition to such effective components as flavonoids, gingko leaves contain ginkgoic acid, bilobol, etc. (these components are present mainly in external seed coat). However, ginkgoic acid, bilobol, ginkgol, etc. have a tendency to cause allergy to some people. It is therefore necessary to reduce the quantities of these substances contained in extracts from ginkgo leaves.

In German Patent Publication No. 1,767,098 is described a process wherein ginkgo leaves are extracted at ca. 40 to 100 °C with a water containing organic solvent miscible with water, and the extract is then extracted with a lipophilic solvent immiscible with water (e.g., carbon tetrachloride), followed by the evaporation of the lipophilic solvent from the water-containing organic extract.

In German Patent Publication No. 2,117,429 is disclosed a process in which ammonium sulfate is added to a water-containing organic extract obtained in accordance with the process described in German Patent Publication No. 1,767,098, the resulting mixture is extracted with methyl ethyl ketone, a lead compound is added to the extract, precipitates formed are removed off, and then the supernatant is extracted with an organic solvent.

In the above-described processes for extracting effective components from ginkgo leaves, there is used an organic halogenoide (e.g., carbon tetrachloride) or methyl ethyl ketone. In case where they are to be used for medical purposes, an additional step is required in order not to allow the organic solvents to remain in the final products. However, in cases where they are to be used for health foods, the above-described extraction and purification processes could not be utilized for their production since it is not allowed to use organic solvents in any of steps for producing health foods.

The inventors have attempted to utilize supercritical carbon dioxide for the purification of effective components contained in powdery extracts obtained by extracting ginkgo leaves with ethanol and then removing the ethanol by evaporation. It was however not possible to remove harmful components at a sufficient rate even when a small quantity of an organic solvent (e.g., ethanol) was added as an entrainer to the supercritical fluid.

It is an object of the invention to enhance the extraction efficiency of the extraction process.

This object is solved by the characterizing features of claim 1.

As a result, it has now been found that the concentration of effective components can be enhanced and at the same time the concentration of harmful substances can be reduced by (i) admixing powdery extracts from ginkgo leaves with ethanol in a ratio where a dispersion is formed and then (ii) introducing supercritical carbon dioxide into the dispersion.

In other words, when a fluid which is in supercritical state is introduced into a dispersion of a solid sample (such as powdery extracts from ginkgo leaves) in an organic solvent, satisfactory results can be obtained even in the case of a solid sample which could hardly be extracted with a good extraction rate by ordinary extraction procedure.

In the extraction process of the invention, ethanol can be added, as an entrainer, to supercritical carbon dioxide so that the state of the dispersion could be maintained as constant as possible.

The powders of extracts from ginkgo leaves can be prepared by extracting ginkgo leaves with ethanol and then removing the solvent therefrom. The powders do not easily dissolve in ethanol. They dissolve in ethanol only in part and the solvent and the powders form a mixture in which the powders are in a dispersed state. In the present invention, such a mixture is referred to as dispersion.

After the extraction procedure has been completed, the ethanol used to disperse the powders can be removed by introducing supercritical carbon dioxide without any trace of ethanol into the dispersion.

In the process of the present invention, there can be used various kinds of supercritical fluids and organic solvents. It can however be preferable for reason of safety to use carbon dioxide and ethanol in cases where the extracts are to be used for foods.

With the above-described constitution, the extraction of the invention is carried out by using supercritical carbon dioxide and without using organic solvents which can be harmful if contained in foods or which could hardly be removed off without difficulty. Accordingly, the process can be of high safety and requires only a simple aftertreatment.

In addition, enhanced extraction rates can be attained even in the case of solid samples which could not be extracted with good results by a simple supercritical extraction.

### Brief Description of the Drawing:

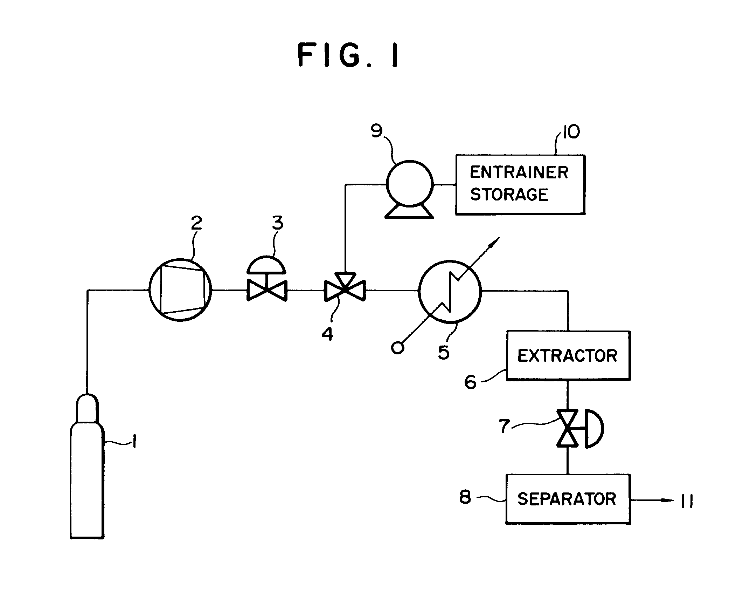

Fig. 1 is a flow sheet of an extraction system usable in the practice of the process according to the invention.

### Best Mode for Carrying Out the Invention:

Carbon dioxide is separated from extracted substances in separation tank 8 and then released into the atmosphere or recycled.

### Example 1

There was prepared a dispersion by admixing 15 ml of ethanol with 1 g crude powders, which was obtained by extracting ginkgo leaves with ethanol and in which were contained 2.8% by weight of effective components and 2.0% by weight of harmful components. The dispersion was subjected to extraction using an extraction system as shown in Fig. 1.

Carbon dioxide is supplied from bomb 1 to compressor 2. After being compressed by the compressor, carbon dioxide flow, via pressure control valve 3 and three-way valve 4, to heat exchanger 5 where it is heated to a predetermined temperature.

Supercritical carbon dioxide having a pressure of 39,2 MPa (400 kg/cm²) at 70 °C and containing 9% by weight of ethanol, from entrainer reservoir 10 pumped by high pressure injection pump 9 through three-way valve 4, was supplied for 30 minutes, and the supercritical fluid flowing out of the extractor is decompressed at pressure reducing valve 7 and then supplied to separator 8, whereby the flow rate of carbon dioxide flowing out of the outlet of the separator 8 was adjusted to 3 1/min.

Thereafter, carbon dioxide not containing ethanol was supplied to the system in order to remove off ethanol contained in the dispersion, and the pressure in the extraction tank was reduced so as to take out the extraction residue.

The quantities of kaempferol and quercetion (effective components) contained in the residue were determined by high performance liquid chromatography, and the quantities of ginkgoic acid and bilobol (harmful substances)were determined by gas chromatography (GC) and GC-mass spectrometry.

In the thus obtained residue were contained 3.8% by weight of effective components and 0.04% by weight of harmful components.

The extraction procedure was repeated in the same manner as above, except that the quantity of ethanol added to carbon dioxide was increased to 20% or 40% by weight. The contents of effective and harmful components contained in the residues showed no substantial differences, and the weight of the residues decreased.

### Comparative Example 1

One gram of powders of the same sample was placed in the extractor tank of the same extraction system as the one used in Example 1 and subjected to the same extraction treatment under the same conditions.

The resulting extraction residue contained 1.96% by weight of harmful components.

### Comparative Examples 2

The extraction procedure was repeated in the same manner as in Comparative Example 1, except that 0.5% or 2% by weight of ethanol was added as an entrainer to carbon dioxide. The resulting residues contained harmful components in an amount of 1.8% or 2% by weight, respectively.

As explained hereinabove, the process of the present invention makes it possible to extract and separate components contained in solid samples in an effective manner, by utilizing a dispersion of solid samples dispersed in an organic solvent. In addition to this, the organic solvent used for the dispersion can be removed off by the use of supercritical fluid.

## Claims

1. A process for extracting and separating substances contained in a solid sample by using a fluid in supercritical state, which comprises contacting the supercritical fluid with the solid sample in order to extract and separate a desired composition from the solid sample,
characterized in that
the solid sample in the form of a powder is dispersed in an organic solvent, that the fluid in supercritical state contains the same organic solvent as the one in which the solid sample is dispersed and that after the extraction procedure fluid in supercritical state is charged into the extractor without any organic solvent so as to remove the organic solvent from the solid sample.

2. A process as defined in claim 1,
wherein said solid sample is an extract from ginkgo leaves and the organic solvent that disperses the solid sample is ethanol.

3. A process as defined in claim 1 or 2,
wherein that supercritical fluid contains an entrainer.

4. A process as defined in any of claims 1 to 3,
wherein said supercritical fluid is carbon dioxide.

5. A process as defined in claims 1, 3 or 4,
wherein the organic solvent is an alcohol.

## Patentansprüche

1. Verfahren zur Extraktion und Trennung von Substanzen, die in einer festen Probe enthalten sind, unter Verwendung eines Fluids in superkritischem Zustand, wobei das superkritische Fluid mit der festen Probe in Kontakt gebracht wird, um eine erwünschte Zusammensetzung aus der festen Probe zu extrahieren und zu trennen,
**dadurch gekennzeichnet**,
daß die feste Probe in der Form eines Pulvers in einem organischen Lösungsmittel dispergiert ist, daß das Fluid in superkritischem Zustand das gleiche organische Lösungsmittel enthält wie jenes, in dem die feste Probe dispergiert ist, und daß nach der Extraktion Fluid in superkritischem Zustand in den Extraktor eingegeben wird, ohne jegliches organisches Lösungsmittel, so daß das organische Lösungsmittel von der festen Probe entfernt wird.

2. Verfahren nach Anspruch 1, wobei die feste Probe ein Extrakt von Ginkgo-Blättern ist und das organische Lösungsmittel Ethanol ist, das die feste Probe fein verteilt.

3. Verfahren nach Anspruch 1 oder 2, wobei das superkritische Fluid ein Schleppmittel enthält.

4. Verfahren nach einem der Ansprüche 1-3, wobei das superkritische Fluid Kohlenstoffdioxid ist.

5. Verfahren nach Anspruch 1, 3 oder 4, wobei das organische Lösungsmittel ein Alkohol ist.

## Revendications

1. Procédé pour l'extraction et la séparation de substances contenues dans un échantillon solide en faisant appel à un fluide à l'état surcritique, procédé qui comprend les opérations consistant à mettre en contact le fluide à l'état surcritique avec l'échantillon solide pour extraire et séparer une composition souhaitée à partir de l'échantillon solide,
caractérisé en ce que
l'échantillon solide sous la forme d'une poudre est mis en dispersion dans un solvant organique, en ce que le fluide à l'état surcritique contient le même solvant organique que celui dans lequel l'échantillon solide est mis en dispersion et en ce qu'après le processus d'extraction, le fluide à l'état surcritique est chargé dans l'extracteur sans aucun solvant organique de façon à faire évacuer le solvant organique de l'échantillon solide.

2. Procédé selon la revendication 1,
dans lequel l'échantillon solide est un extrait de feuille de ginkgo et le solvant organique qui est mis en dispersion dans l'échantillon solide est de l'éthanol.

3. Procédé selon la revendication 1 ou la revendication 2,
dans lequel le fluide à l'état surcritique contient un agent d'entraînement.

4. Procédé selon l'une quelconque des revendications 1 à 3,
dans lequel le fluide à l'état surcritique est du dioxyde de carbone.

5. Procédé selon les revendications 1, 3 ou 4,
dans lequel le solvant organique est un alcool.
